# EUROPEAN PATENT APPLICATION

(11) **EP 2 177 502 A1**
(43) Date of publication of application: **21.04.2010**
(21) Application number: 08166973.1
(22) Date of filing: 17.10.2008
(51) Int. Cl.: C07C 229/10, C07C 237/04, C07D 295/192, A61K 31/165, A61K 31/167, A61K 31/223, A61K 31/5375, A61P 35/00

(54) **Compounds and their use**

(71) Applicant: Oryzon Genomics S.A., 08028 Barcelona (ES)
(72) Inventor: Castro, Julio, 08028 Barcelona (ES)
(74) Representative: ZBM Patents

(57) **Abstract**

Compounds of formula (I) wherein each of R1-R5 is independently selected, may be optionally substituted, and is chosen from -H, halo, alkyl, alkoxy, haloalkyl, haloalkoxy, -L-aryl, -L-heteroaryl, -L-heterocycle, -L-carbocycle, acylamino, acyloxy, alkylthio, alkynyl, amino, aryl, arylalkyl, arylalkenyl, arylalkynyl, arylalkoxy, aryloxy, arylthio, cyano, cyanato, haloaryl, hydroxyl, heteroaryloxy, heteroarylalkoxy, isocyanato, isothiocyanato, nitro, sulfinyl, sulfonyl, sulfonamide, thiocarbonyl, thiocyanato, trihalomethanesulfonamido, O-carbamyl, N-carbamyl, O-thiocarbamyl, N-thiocarbamyl, and C-amido; R6 is chosen from -H and alkyl; R7 is chosen from -H, alkyl, and cycloalkyl; R8 is chosen from -C(=O)NRₓR_{y} and -C(=O)R_{z}; and pharmaceutically acceptable salts thereof, are useful in therapy.

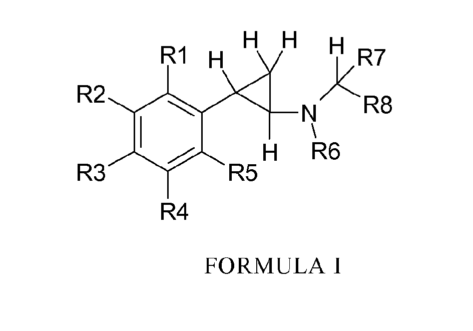

## Description

The invention relates to compounds and their use in therapy.

### BACKGROUND ART

Cancer is prevalent: there were about 3.2 million cancer cases diagnosed (53% men, 47% women) and 1.7 million deaths from cancer (56% men, 44% women) in Europe (Ferlay et al. (2007) Ann. Oncol. 18(3):581-92). In the United States, the probability of developing invasive cancer is 38% for females and 46% for males that live to be 70 years older and older. There will be about 1.4 million new cases of cancer in 2006. Although the five year survival rate for cancer is now 65%, up from about 50% in the mid-nineteen seventies, cancer is deadly. It is estimated that 565,000 people in the United States will die from cancer in 2006 (American Cancer Society, Surveillance Research, 2006). Despite tremendous advances in cancer treatment and diagnosis, cancer remains a major public health concern. Accordingly, there is a need for new therapeutics with activity in cancer.

Another health crisis is facing industrialized nations. As the population in these countries ages, neurodegenerative diseases are affecting more and more people, posing a tremendous economic burden to national health systems. Alzheimer's disease is the largest neurodegenerative disease; disease modifying drugs have long been sought, but to-date, none have been identified. Other neurodegenerative conditions include Parkinson's disease, Huntington's disease, Lewy Body dementia, and which are all characterized by disease progression which robs the patients of their ability to perform normal daily activities, eventually leading to death.

One similar characteristic amongst many cancers and neurodegenerative diseases is aberrant gene expression. A number of compounds have been shown to alter gene expression, including histone deacetylase inhibitors which alter the histone acetylation profile of chromatin. Histone deacetylase inhibitors have been shown to alter gene expression. Another modification that is involved in regulating gene expression is lysine methylation. Methylation of histone lysines has recently been shown to be important in regulating gene expression. A group of enzymes known as lysine demethylases are involved in this histone modification. One particular human histone lysine demethylase enzyme called Lysine Specific Demethylase-1 (LSD1) was recently discovered (Shi et al. (2004) Cell 119:941). LSD1 has a fair degree of structural similarity, and amino acid identity/homology to monoamine oxidases, all of which are flavin dependent oxidases. Recent experiments with LSD1 have shown that it is involved such diverse process as carcinogenesis (Kahl et al. (2006) Cancer Res. 66:1341-11347) and vascular inflammation (Reddy et al. (2008) Circ. Res. 103:615).

It was found that a commercially available antidepressant, Parnate®, which targets monoamine oxidase (MAO), also inhibits LSD1 at clinically relevant concentrations (Lee et al. (2006) Chem. Biol. 13:563-567). Parnate is part of a class of compounds known as phenylcyclopropylamines which are related to another group of clinical useful MAO inhibitors called propargylamines, exemplified by Pargyline which also inhibits LSD1. Additionally, derivatives of Parnate also can inhibit LSD1 (Gooden et al. (2008) Bioorg. Med. Chem. Let. 18:3047-3051). Another other class of compounds was recently disclosed to inhibit LSD1 activity: polyamines (Huang et al. (2007) PNAS 104:8023-8028). The polyamines inhibit LSD1 modestly.

The phenylcyclopropylamines have been the subject of many studies designed to elucidate an SAR for MAO inhibition and determine selectivity for MAO A versus MAO B. Burger et al. ((1962) J. Med. Chem. 5:1243-1265) and Zirkle et al. ((1962) J. Med. Chem. 1265-1284) have disclosed the synthesis and activity of a number of phenylcyclopropylamine related compounds. Zirkle et al. ((1962) J. Med. Chem. 1265-1284) reported that mono- and disubstitution of the amino group of trans-2-phenylcyclopropylamine with methyl decreases the activity only slightly whereas monosubstitution with larger groups like alkyl and araalkyl groups results in considerable loss of activity in the tryptamine potentiation assay for MAO activity. Gooden et al. ((2008) Bioorg. Med. Chem. Let. 18:3047-3051) describe the synthesis of phenylcyclopropylamines derivatives and analogs as well as their activity against MAO A, MAO B, and LSD1. None of the compound made in Gooden et al. showed a lower Ki for LSD1 as compared to either MAO A or MAO B. Additionally, most of the Gooden et al. phenylcyclopropylamine derivatives had increased ability to inhibit MAO A.

In view of the lack of adequate treatments for conditions such as cancer and neurodegeneration, there is a desperate need for disease modifying drugs and drugs that work by inhibiting novel targets. There is a need for the development of LSD1 selective inhibitors as well as dual inhibitors of LSD1 and the monoamine oxidases, particularly those which inhibit LSD1 and MAO B.

### SUMMARY OF THE INVENTION

The present invention relates to the identification of compounds and their use in treated diseases. The present invention provides compounds of Formula I, pharmaceutical compositions comprising a compound of Formula I and a pharmaceutically acceptable carrier, and their use for treating diseases. One use of the compounds of Formula I is to treat cancer.

In one embodiment, the invention provides compounds of Formula I: 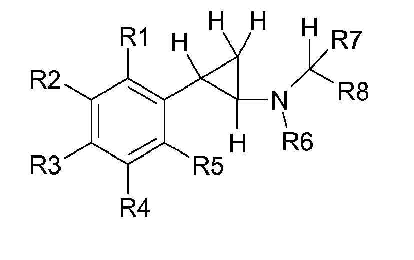 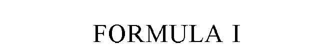 wherein
each of R1-R5 is independently selected, may be optionally substituted, and is chosen from -H, halo, alkyl, alkoxy, haloalkyl, haloalkoxy, -L-aryl, -L-heteroaryl, -L-heterocycle, -L-carbocycle, acylamino, acyloxy, alkylthio, alkynyl, amino, aryl, arylalkyl, arylalkenyl, arylalkynyl, arylalkoxy, aryloxy, arylthio, cyano, cyanato, haloaryl, hydroxyl, heteroaryloxy, heteroarylalkoxy, isocyanato, isothiocyanato, nitro, sulfinyl, sulfonyl, sulfonamide, thiocarbonyl, thiocyanato, trihalomethanesulfonamido, O-carbamyl, N-carbamyl, O-thiocarbamyl, N-thiocarbamyl, and C-amido;
R6 is chosen from -H and alkyl;
R7 is chosen from -H, alkyl, and cycloalkyl;
R8 is chosen from -C(=O)NRₓR_{y} and -C(=O)R₂;
Rₓ is chosen from -H, alkyl, alkynyl, alkenyl, -L-carbocycle, -L-aryl, and -L-heterocycle, all of which may be optionally substituted (except -H);
R_{y} is chosen from -H, alkyl, alkynyl, alkenyl, -L-carbocycle, -L-aryl, and -L-heterocycle, all of which may be optionally substituted (except -H);
R_{z} is chosen from -H, alkoxy, -L-carbocyclic, -L-heterocyclic, -L-aryl, wherein the aryl, heteroaryl, heterocycle, or carbocycle may be optionally substituted;
each L can be saturated, partially saturated, or unsaturated, and is independently chosen from -(CH₂)ₙ-(CH₂)ₙ-, -(CH₂)ₙC(=O)(CH₂)ₙ-,-(CH₂)ₙC(=O)N(CH₂)ₙ-,- (CH₂)ₙNC(=O)O(CH₂)ₙ-, -(CH₂)ₙNC(=O)N(CH₂)ₙ-, - (CH₂)ₙNC(=S)S(CH₂)ₙ-,- (CH₂)ₙOC(O)S(CH₂)ₙ-, -(CH₂)ₙNH(CH₂)ₙ-,-(CH₂)ₙO(CH₂)ₙ-, -(CH₂)ₙS(CH₂)ₙ-, and -(CH₂)ₙNC(=S)N(CH₂)ₙ-, where each n is independently chosen from 0, 1 , 2, 3, 4, 5, 6, 7, and 8, and wherein each carbon and/or nitrogen can be optionally substituted with one or more substituents independently chosen from hydroxyl, halo, alkoxy, alkyl, amino, cycloalkyl, -NRₐR_{b}, -NSO₂R_{c}, -NC(=O)NRₐR_{b}, heteroaryl, aryl, carbocycle, and heterocyclic; wherein -Rₐ and -R_{b} are independently chosen from hydro, alkyl, and -C(=O)OR_{c}; and wherein R_{c} is an alkyl group (e.g., methyl, ethyl, propyl, isopropyl, butyl, isobutyl, or tert-butyl); and pharmaceutically acceptable salts thereof.

Unless otherwise specified each L and each n in a molecule is independently chosen and can be in either orientation, e.g., -(CH₂)ₙNC(=S)S(CH₂)ₙ-, refers to phenylcyclopropylamine-(CH₂)ₙNC(=S)S(CH₂)ₙ-heterocycle and phenylcyclopropylamine-(CH₂)ₙSC(=S)N(CH₂)ₙ-heterocycle orientations.

In one embodiment, the invention provides compounds of Formula I Wherein:
each of R1-R5 is independently selected, may be optionally substituted, and is chosen from -H, halo, alkyl, alkoxy, haloalkyl, haloalkoxy, -L-aryl, -L-heteroaryl, -L-heterocycle, -L-carbocycle, acylamino, acyloxy, alkylthio, alkynyl, amino, aryl, arylalkyl, arylalkenyl, arylalkynyl, arylalkoxy, aryloxy, arylthio, cyano, cyanato, haloaryl, hydroxyl, heteroaryloxy, heteroarylalkoxy, isocyanato, isothiocyanato, nitro, sulfinyl, sulfonyl, sulfonamide, thiocarbonyl, thiocyanato, trihalomethanesulfonamido, O-carbamyl, N-carbamyl, O-thiocarbamyl, N-thiocarbamyl, and C-amido;
R6 is chosen from -H and C1-C6 alkyl;
R7 is chosen from -H, alkyl, and cycloalkyl;
R8 is -C(=O)NRₓR_{y};
Rₓ is chosen from -H, C1-C6 alkyl, C2-C6 alkynyl, C2-C6 alkenyl, -L-carbocycle, -L-aryl, -L-heterocycle, all of which can be optionally substituted (except -H);
R_{y} is chosen from -H, C1-C6 alkyl, C2-C6 alkynyl, C2-C6 alkenyl, -L-carbocycle, -L-aryl, -L-heterocycle, all of which can be optionally substituted (except -H);
each L is independently chosen and is defined as above;
and pharmaceutically acceptable salts thereof.

In one embodiment, the invention provides compounds of Formula I:
wherein
each of R1-R5 is independently selected, may be optionally substituted, and is chosen from -H, halo, alkyl, alkoxy, haloalkyl, haloalkoxy, -L-aryl, -L-heteroaryl, -L-heterocycle, -L-carbocycle, acylamino, acyloxy, alkylthio, alkynyl, amino, aryl, arylalkyl, arylalkenyl, arylalkynyl, arylalkoxy, aryloxy, arylthio, cyano, cyanato, haloaryl, hydroxyl, heteroaryloxy, heteroarylalkoxy, isocyanato, isothiocyanato, nitro, sulfinyl, sulfonyl, sulfonamide, thiocarbonyl, thiocyanato, trihalomethanesulfonamido, O-carbamyl, N-carbamyl, O-thiocarbamyl, N-thiocarbamyl, and C-amido;
R6 is chosen from -H and C1-C6 alkyl;
R7 is chosen from -H, alkyl, and cycloalkyl;
R8 is -C(=O)R_{z};
R_{z} is chosen from -H, C1-C6 alkoxy, -L-carbocyclic, -L-heterocyclic, and -L-aryl, all of which can be optionally substituted (except -H);
each L is independently chosen and is defined as above;
and pharmaceutically acceptable salts thereof.

In one embodiment, the invention provides compounds of Formula I wherein:
each of R1-R5 is independently selected, may be optionally substituted, and is chosen from hydroxyl, halo, alkyl, alkoxy, haloalkyl, haloalkoxy, -N(C₁₋₃ alkyl)₂, -NH(C₁₋₃ alkyl), -C(=O)NH₂, -C(O)NH(C₁₋₃ alkyl), - C(=O)N(C₁₋₃ alkyl) -S(=O)₂(C₁₋₃alkyl), -S(=O)₂NH₂, -S(O)₂N(C₁₋₃ alkyl)₂, -S(=O)₂NH(C₁₋₃ alkyl), -CHF₂ -OCF₃, -OCHF₂ -SCF₃, -CF₃, -CN,-NH₂ and -NO₂;
R6 is chosen from -H and C1-C6 alkyl;
R7 is chosen from -H, alkyl, and cycloalkyl;
R8 is -C(=O)NRₓR_{y};
Rₓ is chosen from -H, C1-C6 alkyl, C2-C6 alkynyl, C2-C6 alkenyl, -L-carbocycle, -L-aryl, -L-heterocycle, all of which can be optionally substituted (except -H);
R_{y} is chosen from -H, C1-C6 alkyl, C2-C6 alkynyl, C2-C6 alkenyl, -L-carbocycle, -L-aryl, -L-heterocycle, all of which can be optionally substituted (except -H);
each L is independently chosen and is defined as above;
and pharmaceutically acceptable salts thereof.

In one embodiment, the invention provides compounds of Formula I wherein:
each of R1-R5 is independently selected, may be optionally substituted, and is chosen from hydroxyl, halo, alkyl alkoxy, halo alkyl haloalkoxy, -N(C₁₋₃ alkyl)₂, -NH(C₁₋₃ alkyl), -C(=O)NH₂, -C(=O)NH(C₁₋₃ alkyl), -C(=O)N(C₁₋₃ alkylb)₂, -S(=O)₂₍C₁₋₃alₖyₗ), -S(-O)₂NH₂, -S(O)₂N(C₁₋₃ alkyl)₂, - S(=O)₂NH(C₁₋₃ alkyl), -CHF₂ -OCF₃, -OCHF₂ -SCF₃, -CF₃, -CN, -NH₂, and -NO_{2;}
R6 is chosen from -H and C1-C6 alkyl;
R7 is chosen from -H, alkyl, and cycloalkyl;
R8 is -C(=O)R_{z};
R_{z} is chosen from -H, -L-carbocyclic, -L-heterocyclic,-L-aryl, wherein the aryl, heterocycle, or carbocycle is optionally substituted;
each L is independently chosen and is defined as above;
and pharmaceutically acceptable salts thereof.

In one embodiment, the invention provides a method of treating a disease or condition comprising administering, to a patient in need of treatment, a therapeutically effectively amount of a composition comprising a compound of Formula I and a pharmaceutically acceptable carrier.

In one embodiment, the invention provides a method of treating a cancer comprising administering, to a patient in need of treatment, a therapeutically effectively amount of a composition comprising a compound of Formula I and a pharmaceutically acceptable carrier.

In one embodiment, the invention provides a method of inhibiting LSD1 activity comprising administering, to a patient in need of treatment, an amount of a composition comprising a compound of Formula I and a pharmaceutically acceptable carrier sufficient to inhibit LSD1 activity.

In one embodiment, the invention provides a method of treating a neurodegenerative disease or disorder comprising administering, to a patient in need of treatment, a therapeutically effectively amount of a composition comprising a compound of Formula I and a pharmaceutically acceptable carrier.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

Other features and advantages of the invention will be apparent from the following detailed description, and from the claims.

### DETAILED DESCRIPTION OF THE INVENTION

The present invention relates to the identification of compounds and their use in treated diseases. The present invention provides compounds of Formula I, pharmaceutical compositions comprising a compound of Formula I and a pharmaceutically acceptable carrier, and their use for treating diseases. One use of the compounds of Formula I is to treat cancer.

In one embodiment, the invention provides compounds of Formula I: 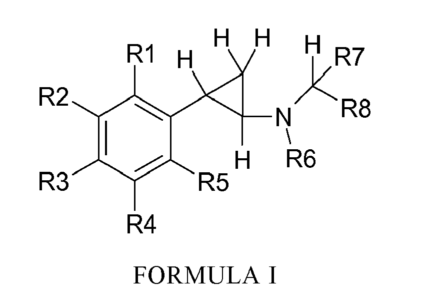 wherein
each of R1-R5 is independently selected, may be optionally substituted, and is chosen from -H, halo, alkyl, alkoxy, haloalkyl, haloalkoxy, -L-aryl, -L-heteroaryl, -L-heterocycle, -L-carbocycle, acylamino, acyloxy, alkylthio, alkynyl, amino, aryl, arylalkyl, arylalkenyl, arylalkynyl, arylalkoxy, aryloxy, arylthio, cyano, cyanato, haloaryl, hydroxyl, heteroaryloxy, heteroarylalkoxy, isocyanato, isothiocyanato, nitro, sulfinyl, sulfonyl, sulfonamide, thiocarbonyl, thiocyanato, trihalomethanesulfonamido, O-carbamyl, N-carbamyl, O-thiocarbamyl, N-thiocarbamyl, and C-amido;
R6 is chosen from -H and alkyl;
R7 is chosen from -H, alkyl, and cycloalkyl;
R8 is chosen from -C(=O)NRₓR_{y} and -C(=O)R_{z};
Rₓ is chosen from -H, alkyl, alkynyl, alkenyl, -L-carbocycle, -L-aryl, -L-heterocycle, all of which can be optionally substituted (except -H);
R_{y} is chosen from -H, alkyl, alkynyl, alkenyl, -L-carbocycle, -L-aryl, -L-heterocycle, all of which can be optionally substituted (except -H);
R_{z} is chosen from -H, alkoxy, -L-carbocyclic, -L-heterocyclic, -L-aryl, wherein the aryl, heteroaryl, heterocycle, or carbocycle is optionally substituted;
each L can be saturated, partially saturated, or unsaturated, and is independently chosen from -(CH₂)ₙ-(CH₂)ₙ-, -(CH₂)ₙC(=O)(CH₂)ₙ-,-(CH₂)ₙC(=O)N(CH₂)ₙ-,- (CH₂)ₙNC(=O)O(CH₂)ₙ-, -(CH₂)ₙNC(=O)N(CH₂)ₙ-,-(CH₂)ₙNC(=S)S(CH₂)ₙ-,- (CH₂)ₙOC(=O)S(CH₂)ₙ-, -(CH₂)ₙNH(CH₂)ₙ-,-(CH₂)ₙO(CH₂)ₙ-, -(CH₂)ₙS(CH₂)ₙ-, and -(CH₂)ₙNC(=S)N(CH₂)ₙ-, where each n is independently chosen from 0, 1 , 2, 3, 4, 5, 6, 7, and 8, and wherein each carbon and/or nitrogen can be optionally substituted with one or more substituents independently chosen from hydroxyl, halo, alkoxy, alkyl, amino, cycloalkyl, -NRₐR_{b}, -NSO₂R_{c}, -NC(=O)NRₐR_{b}, heteroaryl, aryl, carbocycle, and heterocyclic; wherein -Rₐ and -R_{b} are independently chosen from hydro, alkyl, and -C(=O)OR_{c}; and wherein R_{c} is an alkyl group (e.g., methyl, ethyl, propyl, isopropyl, butyl, isobutyl, or tert-butyl); and pharmaceutically acceptable salts thereof.

Unless otherwise specified each L and n in a molecule is independently chosen and can be in either orientation, e.g., -(CH₂)ₙNC(=S)S(CH₂)ₙ-, refers to phenylcyclopropylamine-(CH₂)ₙNC(=S)S(CH₂)ₙ-heterocycle and phenylcyclopropylamine-(CH₂)ₙSC(=S)N(CH₂)ₙ-heterocycle orientations.

In one aspect of this embodiment, each of R1-R5 is independently chosen from -H, halo, C1-C4 alkyl, C-C4 alkoxyl, C1-C4 haloalkyl, -OCH₂(phenyl), and C1-C4 haloalkoxy. In a more specific aspect, each of R1-R5 is independently chosen from -H, halo, -OCH₂(phenyl) and -CF₃. In a more specific aspect each of R1-R5 is-H.

In another aspect of this embodiment, R6 is -H or a C1-C4 alkyl. In a more specific aspect, R6 is -H.

In yet another aspect of this embodiment, R7 is -H or a C1-C4 alkyl. In a more specific aspect, R7 is -H.

In another aspect of this embodiment, each L is chosen from a bond, -CH₂-, -CH₂CH₂-, and -CH₂CH₂CH₂- In a more specific aspect, L is chosen from a bond and -CH₂-.

In another aspect of this embodiment, Rₓ if present, is chosen from -C1-C6 alkyl, C2-C6 alkenyl, C2-C6 alkynyl, -L-cycloalkyl, -L-aryl, -L-heterocycle, wherein the cycloalkyl, aryl, and heterocycle can be optionally substituted. In a more specific aspect, Rₓ is chosen from -H, -CH(CH₃)₂, -C(CH₃)₃, -CH₂C≡CH, -CH₂CH=CH₂, cyclopropyl, cyclobutyl, cyclopentyl, cyclohexyl, and -CH₂(phenyl) wherein the cycloalkyl and phenyl groups can be optionally substituted.

In another aspect of this embodiment, R_{y} if present, is chosen from -C1-C6 alkyl, C2-C6 alkenyl, C2-C6 alkynyl, -L-cycloalkyl, -L-aryl, -L-heterocycle, wherein the cycloalkyl, aryl, and heterocycle can be optionally substituted. In a more specific aspect, R_{y} is chosen from -H, -CH(CH₃)₂, -C(CH₃)₃, -CH₂C≡CH, -CH₂CH=CH₂, cyclopropyl, cyclobutyl, cyclopentyl, cyclohexyl, and -CH₂(phenyl), wherein the cycloalkyl and phenyl groups can be optionally substituted.

In another aspect of this embodiment, R_{z} if present, is an optionally substituted -L-heterocycle. In a more specific aspect, R_{z} is optionally substituted and chosen from N-methylpiperazinyl, morpholinyl, and piperidinyl. In a more specific aspect, R_{z} is chosen from N-methylpiperazinyl, morpholinyl, and piperidinyl.

In one embodiment, the invention provides compounds of Formula I wherein: each of R1-R5 is independently selected, may be optionally substituted, and is chosen from -H, halo, alkyl, alkoxy, haloalkyl, haloalkoxy, -L-aryl, -L-heteroaryl, -L-heterocycle, -L-carbocycle, acylamino, acyloxy, alkylthio, alkynyl, amino, aryl, arylalkyl, arylalkenyl, arylalkynyl, arylalkoxy, aryloxy, arylthio, cyano, cyanato, haloaryl, hydroxyl, heteroaryloxy, heteroarylalkoxy, isocyanato, isothiocyanato, nitro, sulfinyl, sulfonyl, sulfonamide, thiocarbonyl, thiocyanato, trihalomethanesulfonamido, O-carbamyl, N-carbamyl, O-thiocarbamyl, N-thiocarbamyl, and C-amido;
R6 is chosen from -H and C1-C6 alkyl;
R7 is chosen from -H, alkyl, halo, and cycloalkyl;
R8 is -C(=O)NRₓR_{y};
Rₓ is chosen from -H, C1-C6 alkyl, C2-C6 alkynyl, C2-C6 alkenyl, -L-carbocycle, -L-aryl, and -L-heterocycle, all of which can be optionally substituted (except -H);
R_{y} is chosen from -H, C1-C6 alkyl, C2-C6 alkynyl, C2-C6 alkenyl, -L-carbocycle, -L-aryl, and -L-heterocycle, all of which can be optionally substituted (except -H);
each L is independently chosen and is defined as above;
and pharmaceutically acceptable salts thereof.

In one aspect of this embodiment, each of R1-R5 is independently chosen from -H, halo, C1-C4 alkyl, C-C4 alkoxyl, C1-C4 haloalkyl, -OCH₂(phenyl), and C1-C4 haloalkoxy. In a more specific aspect, each of R1-R5 is independently chosen from -H, halo, -OCH₂(phenyl), and -CF₃. In a more specific aspect each of R1-R5 is-H.

In another aspect of this embodiment, R6 is -H or a C1-C4 alkyl. In a more specific aspect, R6 is -H.

In yet another aspect of this embodiment, R7 is -H or a C1-C4 alkyl. In a more specific aspect, R7 is -H.

In another aspect of this embodiment, each L is chosen from a bond,
-CH₂-, -CH₂CH₂-, and - CH₂CH₂CH₂-. In a more specific aspect, L is chosen from a bond and -CH₂-.

In another aspect of this embodiment, Rₓ if present, is chosen from -H, -Cl-C6 alkyl, C2-C6 alkenyl, C2-C6 alkynyl, -L-cycloalkyl, -L-aryl, -L-heterocycle, wherein the cycloalkyl, aryl, and heterocycle can be optionally substituted. In a more specific aspect, Rₓ is chosen from -H, -CH(CH₃)₂, -C(CH₃)₃, -CH₂C≡CH, -CH₂CH=CH₂, cyclopropyl, cyclobutyl, cyclopentyl, cyclohexyl, and -CH₂(phenyl), wherein the cycloalkyl and phenyl groups can be optionally substituted.

In another aspect of this embodiment, R_{y} if present, is chosen from -H, -C1-C6 alkyl, C2-C6 alkenyl, C2-C6 alkynyl, -L-cycloalkyl, -L-aryl, -L-heterocycle, wherein the cycloalkyl, aryl, and heterocycle can be optionally substituted. In a more specific aspect, R_{y} is chosen from -H, -CH(CH₃)₂, -C(CH₃)₃, -CH₂C=CH, -CH₂CH=CH₂, cyclopropyl, cyclobutyl, cyclopentyl, cyclohexyl, and -CH₂(phenyl), wherein the cycloalkyl and phenyl groups can be optionally substituted.

In one embodiment, the invention provides compounds of Formula I wherein: each of R1-R5 is independently selected, may be optionally substituted, and is chosen from -H, halo, alkyl, alkoxy, haloalkyl, haloalkoxy, -L-aryl, -L-heteroaryl, -L-heterocycle, -L-carbocycle, acylamino, acyloxy, alkylthio, alkynyl, amino, aryl, arylalkyl, arylalkenyl, arylalkynyl, arylalkoxy, aryloxy, arylthio, cyano, cyanato, haloaryl, hydroxyl, heteroaryloxy, heteroarylalkoxy, isocyanato, isothiocyanato, nitro, sulfinyl, sulfonyl, sulfonamide, thiocarbonyl, thiocyanato, trihalomethanesulfonamido, O-carbamyl, N-carbamyl, O-thiocarbamyl, N-thiocarbamyl, and C-amido;
R6 is chosen from -H and C1-C6 alkyl;
R7 is chosen from -H, alkyl, and cycloalkyl;
R8 is -C(=O)R_{z};
R_{z} is chosen from -H, -L-carbocyclic, -L-heterocyclic, -L-aryl, wherein the aryl, heterocycle, or carbocycle is optionally substituted;
each L is independently chosen and is defined as above;
and pharmaceutically acceptable salts thereof.

In one aspect of this embodiment, each of R1-R5 is independently chosen from -H, halo, C1-C4 alkyl, C-C4 alkoxyl, C1-C4 haloalkyl, -OCH₂(phenyl), and C1-C4 haloalkoxy. In a more specific aspect, each of R1-R5 is independently chosen from -H, halo, -OCH₂(phenyl), and -CF₃. In a more specific aspect each of R1-R5 is -H.

In another aspect of this embodiment, R6 is -H or a C1-C4 alkyl. In a more specific aspect, R6 is -H.

In yet another aspect of this embodiment, R7 is -H or a C1-C4 alkyl. In a more specific aspect, R7 is -H.

In another aspect of this embodiment, each L is chosen from a bond, -CH₂-, -CH₂CH₂-, and - CH₂CH₂CH₂-. In a more specific aspect, L is chosen from a bond and -CH₂-.

In another aspect of this embodiment, R_{z} if present is an optionally substituted -L-heterocycle. In a more specific aspect, R_{z} is optionally substituted and chosen from N-methylpiperazinyl, morpholinyl, and piperidinyl. In a more specific aspect, R_{z} is chosen from N-methylpiperazinyl, morpholinyl, and piperidinyl.

In one embodiment, the invention provides compounds of Formula I wherein:
each of R1-R5 is independently selected, may be optionally substituted, and is chosen from hydroxyl, halo, alkyl, alkoxy, haloalkyl, haloalkoxy, -N(C₁₋₃ aLkyl)₂, -NH(C₁₋₃ alkyl), -C(=O)NH₂, -C(=O)NH(C₁₋₃ alkyl); - C(=O)N(C₁₋₃ alkyl)₂, -S(-O)₂(C₁₋₃alkyl), -SC(=O)₂NH₂, -S(O)₂N(C₁₋₃ alkyl)₂, -S(=O)₂NH(C₁₋₃ alkyl), -CHF₂, -OCF₃, -OCHF₂, -SCF₃, -CF₃ -CN, -NH₂, and -NO_{2;}
R6 is chosen from -H and C1-C6 alkyl;
R7 is chosen from -H, alkyl, and cycloalkyl;
R8 is -C(=O)NRₓR_{y};
Rₓ is chosen from -H, C1-C6 alkyl, C2-C6 alkynyl, C2-C6 alkenyl, -L-carbocycle, -L-aryl, -L-heterocycle, all of which can be optionally substituted (except -H);
R_{y} is chosen from -H, C1-C6 alkyl, C2-C6 alkynyl, C2-C6 alkenyl, -L-carbocycle, -L-aryl, -L-heterocycle, all of which can be optionally substituted (except -H);
each L is independently chosen and is defined as above;
and pharmaceutically acceptable salts thereof.

In one aspect of this embodiment, each of R1-R5 is independently chosen from -H, halo, C1-C4 alkyl, C-C4 alkoxyl, C1-C4 haloalkyl, -OCH₂(phenyl), and C1-C4 haloalkoxy and -CF₃. In a more specific aspect, each of R1-R5 is independently chosen from -H, halo, -OCH₂(phenyl), and -CF₃. In a more specific aspect each of each of R1-R5 is -H.

In another aspect of this embodiment, R6 is -H or a C1-C4 alkyl. In a more specific aspect, R6 is -H.

In yet another aspect of this embodiment, R7 is -H or a C1-C4 alkyl. In a more specific aspect, R7 is -H.

In another aspect of this embodiment, each L is chosen from a bond, -CH₂-, -CH₂CH₂-, and - CH₂CH₂CH₂-. In a more specific aspect, L is chosen from a bond and -CH₂-.

In another aspect of this embodiment, Rₓ if present, is chosen from -C1-C6 alkyl, C2-C6 alkenyl, C2-C6 alkynyl, -L-cycloalkyl, -L-aryl, -L-heterocycle, wherein the cycloalkyl, aryl, and heterocycle can be optionally substituted. In a more specific aspect, Rₓ is chosen from -H, -CH(CH₃)₂, -C(CH₃)₃, -CH₂C=CH, -CH₂CH=CH₂, cyclopropyl, cyclobutyl, cyclopentyl, cyclohexyl, and -CH₂(phenyl), wherein the cycloalkyl and phenyl groups can be optionally substituted.

In another aspect of this embodiment, R_{y} if present, is chosen from -C1-C6 alkyl, C2-C6 alkenyl, C2-C6 alkynyl, -L-cycloalkyl, -L-aryl, -L-heterocycle, wherein the cycloalkyl, aryl, and heterocycle can be optionally substituted. In a more specific aspect, R_{y} is chosen from -H, -CH(CH₃)₂, -C(CH₃)₃, -CH₂C≡CH, -CH₂CH=CH₂, cyclopropyl, cyclobutyl, cyclopentyl, cyclohexyl, and -CH₂(phenyl), wherein the cycloalkyl and phenyl groups can be optionally substituted.

In one embodiment, the invention provides compounds of Formula I wherein:
each of R1-R5 is independently selected, may be optionally substituted, and is chosen from hydroxyl, hallo, alkyl, alkoxy, haloalkyl, haloalkoxy, -N(C₁₋₃ alkyl)₂, -NH(C₁₋₃ alkyl), -C(-O)NH₂, -C(=O)NH(C1-3alkyl), - C(=O)N(C₁₋₃ alkyl)₂, -S(=O)₂(C₁₋₃alkyl), -S(=O)₂NH₂, -S(O)₂N(C₁₋₃ alkyl)₂, - S(=O)₂NH(C₁₋₃ alkyl) -CHF₂, -OCF₃, -OCHF₂, -SCF₃, -CF₃, -CN. -NH₂, and -NO₂;
R6 is chosen from -H and C1-C6 alkyl;
R7 is chosen from -H, alkyl, and cycloalkyl;
R8 is -C(=O)R_{z};
R_{z} is chosen from -H, -L-carbocyclic, -L-heterocyclic,-L-aryl, wherein the aryl, heterocycle, or carbocycle is optionally substituted;
each L is independently chosen and is defined as above;
and pharmaceutically acceptable salts thereof.

In one aspect of this embodiment, each of R1-R5 is independently chosen from -H, halo, C1-C4 alkyl, C-C4 alkoxyl, C1-C4 haloalkyl, -OCH₂(phenyl), and C1-C4 haloalkoxy. In a more specific aspect, each of R1-R5 is independently chosen from -H, halo, -OCH₂(phenyl), and -CF₃. In a more specific aspect each of each of R1-R5 is -H.

In another aspect of this embodiment, R6 is -H or a C1-C4 alkyl. In a more specific aspect, R6 is -H.

In yet another aspect of this embodiment, R7 is -H or a C1-C4 alkyl. In a more specific aspect, R7 is -H.

In another aspect of this embodiment, each L is chosen from a bond, -CH₂-, -CH₂CH₂-, and - CH₂CH₂CH₂- In a more specific aspect, L is chosen from a bond and -CH₂-.

In another aspect of this embodiment, R_{z} if present, is an optionally substituted -L-heterocycle. In a more specific aspect, R_{z} is optionally substituted and chosen from N-methylpiperazinyl, morpholinyl, and piperidinyl. In a more specific aspect, R_{z} is chosen from N-methylpiperazinyl, morpholinyl, and piperidinyl.

Optional substituents refers to 1-4 substituents on the parent group and are independently chosen from acylamino, acyloxy, alkenyl, alkoxy, alkyl, alkylthio, alkynyl, amino, aryl, arylalkyl, arylalkenyl, arylalkynyl, arylalkoxy, aryloxy, arylthio, carbocycle, cyano, cyanato, halo, haloalkyl, haloaryl, hydroxyl, heteroaryl, heteroaryloxy, heterocycle, heteroarylalkoxy, isocyanato, isothiocyanato, nitro, sulfinyl, sulfonyl, sulfonamide, thiocarbonyl, thiocyanato, trihalomethanesulfonamido, O-carbamyl, N-carbamyl, O-thiocarbamyl, N-thiocarbamyl, and C-amido. In a more specific aspect optional substituents are independently chosen from hydroxyl, halo, alkyl, alkoxy, haloalkyl, haloalkoxy, -N(C₁₋₃ alkyl)₂, -NH(C₁₋₃ alkyl), -C(=O)NH_{2,} -C(=O)NH(C₁₋₃alkyl), -C(=O)N(C₁₋₃ alkyl)₂, -S(-O)₂(C₁₋₃alkyl), -S(=O)₂NH₂, -S(O)₂N(C₁₋₃ alkyl)₂, -S(=O)₂NH(C₁₋₃ alkyl), -CHF₂, -OCF₃, -OCHF₂, -SCF₃ -CF₃, -CN, -NH₂, and -NO₂.

In some of the embodiments related to Formula I, the compound does not have the structure of the compounds having CAS registration nos. 928314-26-5, 917388-09-1, 825630-21-5, 728873-64-1, and/or 728871-98-5.

In one embodiment, the invention provides a method of treating a disease or condition comprising administering, to a patient in need of treatment, a therapeutically effectively amount of a composition comprising a compound of Formula I and a pharmaceutically acceptable carrier. In one aspect of this embodiment, the disease is cancer or a neurodegenerative disease.

In one embodiment, the invention provides a method of treating a cancer comprising administering, to a patient in need of treatment, a therapeutically effectively amount of a composition comprising a compound of Formula I and a pharmaceutically acceptable carrier. In one aspect of this embodiment, the cancer is prostate cancer

In one embodiment, the invention provides a method of treating a neurodegenerative disease or disorder comprising administering, to a patient in need of treatment, a therapeutically effectively amount of a composition comprising a compound of Formula I and a pharmaceutically acceptable carrier.

### Definitions

As used herein, the term "alkyl" refers to a saturated aliphatic hydrocarbon including straight chain and branched chain groups. In a more specific definition, the alkyl group has 1 to 20 carbon atoms (whenever it appears herein, a numerical range such as "1 to 20" refers to each integer in the given range; e.g., "1 to 20 carbon atoms" means that the alkyl group may consist of 1 carbon atom, 2 carbon atoms, 3 carbon atoms, etc. up to and including 20 carbon atoms). In another more specific definition, it is a medium size alkyl having 1 to 10 carbon atoms. In yet another more specific definition, it is a lower alkyl having 1 to 6 carbon atoms, and even more preferably 1 to 4 carbon atoms. The alkyl group may be substituted or unsubstituted. Unless otherwise defined, when substituted, the substituent group(s) is one or more individually selected from cycloalkyl, aryl, heteroaryl, heteroalicyclic, hydroxy, alkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, O- carboxy, cyanato, isocyanato, thiocyanato, isothiocyanato, nitro, silyl, and amino.

As used herein, the term "halo" refers to chloro, fluoro, bromo, and iodo.

As used herein, the term "hydro" refers to a hydrogen atom (-H group).

As used herein, the term "hydroxy" refers to an -OH group.

As used herein, the term "alkoxy" refers to both an -O-alkyl and an -O-cycloalkyl group, as defined herein. Lower alkoxy refers to -O-lower alkyl groups.

As used herein, the term "aryloxy" refers to both an -O-aryl and an -O-heteroaryl group, as defined herein.

As used herein, the term "mercapto" group refers to an -SH group. As used herein, the term "alkylthio" group refers to both an S-alkyl and an -S-cycloalkyl group, as defined herein.

As used herein, the term "arylthio" group refers to both an -S-aryl and an -S-heteroaryl group, as defined herein.

As used herein, the term "carbonyl" group refers to a -C(=O)R" group, where R" is selected from the group consisting of hydro, alkyl, cycloalkyl, aryl, heteroaryl (bonded through a ring carbon) and heterocyclic (bonded through a ring carbon), as defined herein.

As used herein, the term "aldehyde" group refers to a carbonyl group where R" is hydro.

As used herein, the term "cycloketone" refer to a cycloalkyl group in which one of the carbon atoms which form the ring has a "=O" bonded to it; i.e. one of the ring carbon atoms is a -C(=O)-group.

As used herein, the term "thiocarbonyl" group refers to a -C(=S)R" group, with R" as defined herein.

As used herein, the term "O-carboxy" group refers to a R"C(=O)O-group, with R" as defined herein.

As used herein, the term "C-carboxy" group refers to a -C(=O)OR" groups with R" as defined herein.

As used herein, the term "ester" is a C-carboxy group, as defined herein, wherein R" is any of the listed groups other than hydro.

As used herein, the term "C-carboxy salt" refers to a -C(=O)O⁻M⁺ group wherein M⁺ is selected from the group consisting of lithium, sodium, magnesium, calcium, potassium, barium, iron, zinc and quaternary ammonium.

As used herein, the term "acetyl" group refers to a -(C=O)CH₃ group.

As used herein, the term "carboxyalkyl" refers to -(CH₂)ᵣC(=O)OR" wherein r is 1-6 and R" is as defined above.

As used herein, the term "carboxyalkyl salt" refers to a -(CH₂)ᵣC(=O)O⁻M⁺ wherein M⁺ is selected from the group consisting of lithium, sodium, potassium, calcium, magnesium, barium, iron, zinc and quaternary ammonium.

As used herein, the term "carboxylic acid" refers to a C-carboxy group in which R" is hydro.

As used herein, the term "haloalkyl" refers to an alkyl group substituted with 1 to 6 halo groups. In a specific embodiment, haloalkyl is a -CX₃ group wherein X is a halo group. The halo groups can be independently selected.

As used herein, the term "trihalomethanesulfonyl" refers to a X₃CS(=O)₂-group with X as defined above.

As used herein, the term "cyano" refers to a -C=N group.

As used herein, the term "cyanato" refers to a -CNO group.

As used herein, the term "isocyanato" refers to a -NCO group.

As used herein, the term "thiocyanato" refers to a -CNS group. As used herein, the term "isothiocyanato" refers to a -NCS group.

As used herein, the term "sulfinyl" refers to a -S(=O)R" group, with R" as defined herein.

As used herein, the term "sulfonyl" refers to a -S(=O)₂R" group, with R" as defined herein. As used herein, the term "sulfonamido" refers to a -S(=O)₂ NR₁₇R₁₈, with R₁₇ and R₁₈ as defined herein.

As used herein, the term "trihalomethanesulfonamido" refers to a X₃CS(=O)₂NR₁₇- group with X and R₁₇ as defined herein.

As used herein, the term "O-carbamyl" refers to a -OC(=O)NR₁₇R₁₈ group with R₁₇ and R₁₈ as defined herein. As used herein, the term "N-carbamyl" refers to a R₁₈OC(=O)NR₁₇- group, with R₁₇ and R₁₈ as defined herein.

As used herein, the term "O-thiocarbamyl" refers to a -OC(=S)NR₁₇R₁₈ group with R₁₇ and R₁₈ as defined herein.

As used herein, the term "N-thiocarbamyl" refers to a R₁₇O(C=S)NR₁₈- group, with R₁₇ and R₁₈ as defined herein.

As used herein, the term "amino" refers to an -NRR group, with R and R both being hydro.

As used herein, the term "C-amido" refers to a -C(=0)NR₁₇R₁₈ group with R₁₇ and R₁₈ as defined herein. An "N-amido" refers to a R₁₇C(=0)NR₁₈- group with R17 and R18 as defined herein.

As used herein, the term "nitro" refers to a -NO₂ group.

As used herein, the term "quaternary ammonium" refers to a -NR₁₇R₁₈ R₁₉ group wherein R₁₇, R₁₈, and R₁₉ are independently selected from the group consisting of hydro and unsubstituted lower alkyl.

As used herein, the term "methylenedioxy" refers to a -OCH₂O- group wherein the oxygen atoms are bonded to adjacent ring carbon atoms.

As used herein, the term "ethylenedioxy" refers to a -OCH₂CH₂O- group wherein the oxygen atoms are bonded to adjacent ring carbon atoms.

As used herein, the term "carbocyclic" refers to an all-carbon monocyclic or fused ring (i.e., rings which share an adjacent pair of carbon atoms) group wherein one or more of the rings does not have a completely conjugated pi-electron system. Examples, without limitation, of carbocyclic groups are cycloalkyls such as cyclopropane, cyclobutane, cyclopentane, cyclohexane, adamantane, cycloheptane and cycloalkenes such as cycloheptatriene, cyclopentene, and cyclohexadiene. A carbocyclic group may be substituted or unsubstituted. Unless otherwise specified, when substituted, the substituent group(s) is one or more groups individually selected from alkyl, aryl, heteroaryl, heterocyclic, hydroxy, alkoxy, aryloxy, mercapto, alkylthio, arylthio, cyano, halo, carbonyl, thiocarbonyl, carboxy, O-carbamyl, N-carbamyl, C- amido, N-amido, nitro, and amino.

As used herein, the term "heterocycle" or heterocyclic" refers to a saturated or partially saturated 3-7 membered monocyclic, or 7-10 membered bicyclic ring system, which consists of carbon atoms and from one to four heteroatoms independently selected from the group consisting of O, N, and S, wherein the nitrogen and sulfur heteroatoms can be optionally oxidized, the nitrogen can be optionally quaternized, and including any bicyclic group in which any of the above-defined heterocyclic rings is fused to a benzene ring, and wherein the heterocyclic ring can be substituted on carbon or on a nitrogen atom if the resulting compound is stable. Non- limiting saturated or partially saturated heterocyclic groups include tetrahydrofuranyl, pyranyl, piperidinyl, piperazinyl, pyrrolidinyl, imidazolidinyl, imidazolinyl, indolinyl, isoindolinyl, quinuclidinyl, morpholinyl, isochromanyl, chromanyl, pyrazolidinyl, pyrazolinyl, tetronoyl and tetramoyl groups. Example of "heterocycles" or "heterocyclic" rings also include, but are not limited to, morpholino, piperidyl, piperazinyl, pyrrolidinyl, thiomorpholino, homopiperazinyl, imidazolyl, imidazolidinyl, pyrazolidinyl, dioxanyl and dioxolanyl. "Heterocycle" can include heteroaryls when the pi-electron system of a heterocycle is completely conjugated. The heterocycle group may be substituted or unsubstituted. Unless otherwise defined, when substituted, the substituted group(s) is one or more selected from halo, trihalomethyl, alkyl, hydroxy, alkoxy, aryloxy, mercapto, alkylthio, arylthio, cyano, nitro, carbonyl, thiocarbonyl, C-carboxy, O-carboxy, O-carbamyl, N-carbamyl, O-thiocarbamyl, N-thiocarbamyl, C-amido, N-amido, sulfmyl, sulfonyl, S-sulfonamido, N-sulfonamido, trihalomethanesulfonamido, and amino.

As used herein, the term "aryl" refers to an all-carbon monocyclic or fused-ring polycyclic (i.e., rings which share adjacent pairs of carbon atoms) groups having a completely conjugated pi-electron system. Examples, without limitation, of aryl groups are phenyl, naphthalenyl and anthracenyl. The aryl group may be substituted or unsubstituted. Unless otherwise defined, when substituted, the substituent group(s) is one or more selected from halo, trihalomethyl, alkyl, hydroxy, alkoxy, aryloxy, mercapto, alkylthio, arylthio, cyano, nitro, carbonyl, thiocarbonyl, C-carboxy, O-carboxy, O-carbamyl, N-carbamyl, O-thiocarbamyl, N-thiocarbamyl, C-amido, N-amido, sulfmyl, sulfonyl, S-sulfonamido, N-sulfonamido, trihalomethanesulfonamido, and amino.

As used herein, the term "heteroaryl" refers to groups having 5 to 14 ring atoms; 6, 10 or 14 pi electrons shared in a cyclic array; and containing carbon atoms and 1, 2 or 3 oxygen, nitrogen or sulfur heteroatoms. Non-limiting heteroaryl groups include thienyl (thiophenyl), benzo[b]thienyl, naphtho[2,3-b]thienyl, thianthrenyl, furyl (furanyl), isobenzofuranyl, chromenyl, xanthenyl, phenoxanthiinyl, pyrrolyl, including without limitation 2H-pyrrolyl, imidazolyl, pyrazolyl, pyridyl (pyridinyl), including without limitation 2-pyridyl, 3 -pyridyl, and 4-pyridyl, pyrazinyl, pyrimidinyl, pyridazinyl, indolizinyl, isoindolyl, 3H-indolyl, indolyl, indazolyl, purinyl, 4H-quinolizinyl, isoquinolyl, quinolyl, phthalzinyl, naphthyridinyl, quinozalinyl, cinnolinyl, pteridinyl, carbazolyl, beta-carbolinyl, phenanthridinyl, acrindinyl, perimidinyl, phenanthrolinyl, phenazinyl, isothiazolyl, phenothiazinyl, isoxazolyl, furazanyl, phenoxazinyl, 7-aminoisocoumarin, pyrido[1,2-a]pyrimidin-4-one, pyrazolo[1,5-a]pyrimidinyl, including without limitation pyrazolo[1,5-a]pyrimidin-3-yl, 1,2-benzoisoxazol-3-yl, benzimidazolyl, 2-oxindolyl and 2 oxobenzimidazolyl. When the heteroaryl group contains a nitrogen atom in a ring, such nitrogen atom may be in the form of an N-oxide, e.g., a pyridyl N-oxide, pyrazinyl N-oxide and pyrimidinyl N-oxide. Unless otherwise defined, when substituted, the substituent group(s) is one or more selected from alkyl, cycloalkyl, halo, trihalomethyl, hydroxy, alkoxy, aryloxy, mercapto, alkylthio, arylthio, cyano, nitro, carbonyl, thiocarbonyl, sulfonamido, carboxy, sulfinyl, sulfonyl, O-carbamyl, N-carbamyl, O-thiocarbamyl, N-thiocarbamyl, C-amido, N-amido, and amino.

As used herein, the term "arylalkyl" refers to any of the C₁₋₁₀ alkyl groups substituted by any of the above-mentioned C₆₋₁₄ aryl groups as defined herein. Non-limiting examples of arylalkyl group include benzyl, phenethyl, and naphthylmethyl.

As used herein, the term "arylalkenyl" is used herein to mean any of the above-mentioned C₂₋₁₀ alkenyl groups substituted by any of the above-mentioned C₆₋₁₄ aryl groups.

As used herein, the term "arylalkynyl" refers to any of C₂₋₁₀ alkynyl groups substituted by any of the above-mentioned C₆₋₁₄ aryl groups as defined herein.

As used herein, the term "arylalkoxy" refers to any of the C₁₋₁₀ alkoxy groups substituted by any of the aryl groups as defined herein, which may be optionally substituted. Examples of arylalkoxy groups include benzyloxy and phenethyloxy.

As used herein, the term "aryloxy" refers to oxygen substituted by any of the C₆₋₁₄ aryl groups defined herein, which may be optionally substituted. Examples of aryloxy groups include phenoxy and 4-methylphenoxy.

As used herein, the term "arylthio" group refers to both an -S-aryl and an -S-heteroaryl group, as defined herein.

As used herein, the term "preventing an increase in a symptom" refers to both not allowing a symptom to increase or worsen, as well as reducing the rate of increase in the symptom. For example, a symptom can be measured as the amount of particular disease marker, i.e., a protein. In another example the symptom can be cognitive decline. Preventing an increase, according to the definition provided herein, means that the amount of symptom {e.g., protein or cognitive decline) does not increase or that the rate at which it increases is reduced.

As used herein, the term "treating a disease or disorder" refers to a slowing of or a reversal of the progress of the disease. Treating a disease or disorder includes treating a symptom and/or reducing the symptoms of the disease.

As used herein, the term "preventing a disease or disorder" refers to a slowing of the disease or of the onset of the disease or the symptoms thereof. Preventing a disease or disorder can include stopping the onset of the disease or symptoms thereof. As used herein, the term "unit dosage form" refers to a physically discrete unit, such as a capsule or tablet suitable as a unitary dosage for a human patient. Each unit contains a predetermined quantity of a compound of Formula I, which was discovered or believed to produce the desired pharmacokinetic profile which yields the desired therapeutic effect. The dosage unit is composed of a compound of Formula I in association with at least one pharmaceutically acceptable carrier, salt, excipient, or combination thereof.

As used herein, the term "dose" or "dosage" refers the amount of active ingredient that an individual takes or is administered at one time. For example, a 40 mg dose of a compound of Formula I refers to, in the case of a twice-daily dosage regimen, a situation where the individual takes 40 mg of a compound of Formula I twice a day, e.g., 40 mg in the morning and 40 mg in the evening. The 40 mg of a compound of Formula I dose can be divided into two or more dosage units, e.g., two 20 mg dosage units of a compound of Formula I in tablet form or two 20 mg dosage units of a compound of Formula I in capsule form.

As used herein, a "pharmaceutically acceptable prodrug" is a compound that may be converted under physiological conditions or by solvolysis to the specified compound or to a pharmaceutically acceptable salt of such compound.

As used herein, a "pharmaceutically active metabolite" is intended to mean a pharmacologically active product produced through metabolism in the body of a specified compound or salt thereof. Metabolites of a compound may be identified using routine techniques known in the art and their activities determined using tests such as those described herein.

As used herein, a "pharmaceutically acceptable salt" is intended to mean a salt that retains the biological effectiveness of the free acids and bases of the specified compound and that is not biologically or otherwise undesirable. A compound for use in the invention may possess a sufficiently acidic, a sufficiently basic, or both functional groups, and accordingly react with any of a number of inorganic or organic bases, and inorganic and organic acids, to form a pharmaceutically acceptable salt. Exemplary pharmaceutically acceptable salts include those salts prepared by reaction of the compounds of the present invention with a mineral or organic acid or an inorganic base, such as salts including sulfates, pyrosulfates, bisulfates, sulfites, bisulfites, phosphates, monohydrophosphates, dihydrophosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4 dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, gamma-hydroxybutyrates, glycollates, tartrates, methane-sulfonates, propanesulfonates, naphthalene-1-sulfonates, naphthalene-2-sulfonates, and mandelates.

As used herein, a "pharmaceutically acceptable carrier" refers to a non-API (API refers to Active Pharmaceutical Ingredient) substances such as disintegrators, binders, fillers, and lubricants used in formulating pharmaceutical products. They are generally safe for administering to humans according to established governmental standards, including those promulgated by the United States Food and Drug Administration and the European Medical Agency.

As is understood by the skilled artisan, certain variables in the list of substituents are repetitive (different name for the same substituent), generic to other terms in the list, and/or partially overlap in content with other terms. In the compounds of the invention, the skilled artisan recognizes that substituents may be attached to the remainder of the molecule via a number of positions and the preferred positions are as illustrated in the Examples.

Additionally, the compounds of Formula I can contain asymmetric carbon atoms and can therefore exist in racemic and optically active forms. Thus, optical isomers or enantiomers, racemates, tautomers, and diastereomers are also encompassed in the compounds of Formula I. The methods of present invention include the use of all such isomers and mixtures thereof. Methods of separation of enantiomeric and diastereomeric mixtures are well known to one skilled in the art. The present invention encompasses any isolated racemic or optically active form of compounds described in Formula I, or any mixture thereof. In one aspect, the compounds of the invention have a trans configuration around the cyclopropyl ring as in trans-phenylcyclopropylamine. In one aspect, the compounds of the invention have a cis configuration around the cyclopropyl ring as in cis-phenylcyclopropylamine.

Typically, compounds according to Formula I can be effective at an amount of from about 0.01 µg/kg to about 100 mg/kg per day based on total body weight. The active ingredient may be administered at once, or may be divided into a number of smaller doses to be administered at predetermined intervals of time. The suitable dosage unit for each administration can be, e.g., from about 1 µg to about 2000 mg, preferably from about 5 µg to about 1000 mg.

It should be understood that the dosage ranges set forth above are exemplary only and are not intended to limit the scope of this invention. The therapeutically effective amount for each active compound can vary with factors including but not limited to the activity of the compound used, stability of the active compound in the patient's body, the severity of the conditions to be alleviated, the total weight of the patient treated, the route of administration, the ease of absorption, distribution, and excretion of the active compound by the body, the age and sensitivity of the patient to be treated, and the like, as will be apparent to a skilled artisan. The amount of administration can be adjusted as the various factors change over time.

For oral delivery, the active compounds can be incorporated into a formulation that includes pharmaceutically acceptable carriers such as binders (e.g., gelatin, cellulose, gum tragacanth), excipients (e.g., starch, lactose), lubricants (e.g., magnesium stearate, silicon dioxide), disintegrating agents (e.g., alginate, Primogel, and corn starch), and sweetening or flavoring agents (e.g., glucose, sucrose, saccharin, methyl salicylate, and peppermint). The formulation can be orally delivered in the form of enclosed gelatin capsules or compressed tablets. Capsules and tablets can be prepared in any conventional techniques. The capsules and tablets can also be coated with various coatings known in the art to modify the flavors, tastes, colors, and shapes of the capsules and tablets. In addition, liquid carriers such as fatty oil can also be included in capsules.

Suitable oral formulations can also be in the form of suspension, syrup, chewing gum, wafer, elixir, and the like. If desired, conventional agents for modifying flavors, tastes, colors, and shapes of the special forms can also be included. In addition, for convenient administration by enteral feeding tube in patients unable to swallow, the active compounds can be dissolved in an acceptable lipophilic vegetable oil vehicle such as olive oil, corn oil and safflower oil.

The active compounds can also be administered parenterally in the form of solution or suspension, or in lyophilized form capable of conversion into a solution or suspension form before use. In such formulations, diluents or pharmaceutically acceptable carriers such as sterile water and physiological saline buffer can be used. Other conventional solvents, pH buffers, stabilizers, anti-bacteria agents, surfactants, and antioxidants can all be included. For example, useful components include sodium chloride, acetates, citrates or phosphates buffers, glycerin, dextrose, fixed oils, methyl parabens, polyethylene glycol, propylene glycol, sodium bisulfate, benzyl alcohol, ascorbic acid, and the like. The parenteral formulations can be stored in any conventional containers such as vials and ampoules.

Routes of topical administration include nasal, bucal, mucosal, rectal, or vaginal applications. For topical administration, the active compounds can be formulated into lotions, creams, ointments, gels, powders, pastes, sprays, suspensions, drops and aerosols. Thus, one or more thickening agents, humectants, and stabilizing agents can be included in the formulations. Examples of such agents include, but are not limited to, polyethylene glycol, sorbitol, xanthan gum, petrolatum, beeswax, or mineral oil, lanolin, squalene, and the like. A special form of topical administration is delivery by a transdermal patch. Methods for preparing transdermal patches are disclosed, e.g., in Brown, et al. (1988) Ann. Rev. Med. 39:221-229 which is incorporated herein by reference.

Subcutaneous implantation for sustained release of the active compounds may also be a suitable route of administration. This entails surgical procedures for implanting an active compound in any suitable formulation into a subcutaneous space, e.g., beneath the anterior abdominal wall. See, e.g., Wilson et al. (1984) J. Clin. Psych. 45:242-247. Hydrogels can be used as a carrier for the sustained release of the active compounds. Hydrogels are generally known in the art. They are typically made by crosslinking high molecular weight biocompatible polymers into a network, which swells in water to form a gel like material. Preferably, hydrogels are biodegradable or biosorbable. For purposes of this invention, hydrogels made of polyethylene glycols, collagen, or poly(glycolic-co-L-lactic acid) may be useful. See, e.g., Phillips et al. (1984) J. Pharmaceut. Sci., 73: 1718-1720.

The active compounds can also be conjugated, to a water soluble non-immunogenic non-peptidic high molecular weight polymer to form a polymer conjugate. For example, an active compound is covalently linked to polyethylene glycol to form a conjugate. Typically, such a conjugate exhibits improved solubility, stability, and reduced toxicity and immunogenicity. Thus, when administered to a patient, the active compound in the conjugate can have a longer half-life in the body, and exhibit better efficacy. See generally, Burnham (1994) Am. J. Hosp. Pharm. 15:210-218. PEGylated proteins are currently being used in protein replacement therapies and for other therapeutic uses. For example, PEGylated interferon (PEG-INTRON A®) is clinically used for treating Hepatitis B. PEGylated adenosine deaminase (ADAGEN®) is being used to treat severe combined immunodeficiency disease (SCIDS). PEGylated L-asparaginase (ONCAPSPAR®) is being used to treat acute lymphoblastic leukemia (ALL). It is preferred that the covalent linkage between the polymer and the active compound and/or the polymer itself is hydrolytically degradable under physiological conditions. Such conjugates known as "prodrugs" can readily release the active compound inside the body. Controlled release of an active compound can also be achieved by incorporating the active ingredient into microcapsules, nanocapsules, or hydrogels generally known in the art. Other pharmaceutically acceptable prodrugs of the compounds of this invention include, but are not limited to, esters, carbonates, thiocarbonates, N-acyl derivatives, N-acyloxyalkyl derivatives, quaternary derivatives of tertiary amines, N-Mannich bases, Schiff bases, aminoacid conjugates, phosphate esters, metal salts and sulfonate esters.

Liposomes can also be used as carriers for the active compounds of the present invention. Liposomes are micelles made of various lipids such as cholesterol, phospholipids, fatty acids, and derivatives thereof. Various modified lipids can also be used. Liposomes can reduce the toxicity of the active compounds, and increase their stability. Methods for preparing liposomal suspensions containing active ingredients therein are generally known in the art. See, e.g., U.S. Patent No. 4,522,81 1 ; Prescott, Ed., Methods in Cell Biology, Volume XIV, Academic Press, New York, N. Y. (1976).

The active compounds can also be administered in combination with another active agent that synergistically treats or prevents the same symptoms or is effective for another disease or symptom in the patient treated so long as the other active agent does not interfere with or adversely affect the effects of the active compounds of this invention. Such other active agents include but are not limited to anti-inflammation agents, antiviral agents, antibiotics, antifungal agents, antithrombotic agents, cardiovascular drugs, cholesterol lowering agents, anti-cancer drugs, hypertension drugs, and the like.

Examples of antineoplastic agents that can be used in combination with the compounds and methods of the present invention include, in general, and as appropriate, alkylating agents, anti-metabolites, epidophyllotoxins, antineoplastic enzymes, topoisomerase inhibitors, procarbazines, mitoxantrones, platinum coordination complexes, biological response modifiers and growth inhibitors, hormonal/anti-hormonal therapeutic agents and haematopoietic growth factors. Exemplary classes of antineoplastic include the anthracyclines, vinca drugs, mitomycins, bleomycins, cytotoxic nucleosides, epothilones, discodermolides, pteridines, diynenes and podophyllotoxins. Particularly useful members of those classes include, for example, carminomycin, daunorubicin, aminopterin, methotrexate, methopterin, dichloromethotrexate, mitomycin C, porfiromycin, 5- fluorouracil, 6-mercaptopurine, gemcitabine, cytosine arabinoside, podophyllotoxin or podophyllotoxin derivatives such as etoposide, etoposide phosphate or teniposide, melphalan, vinblastine, vincristine, leurosidine, vindesine, leurosine, paclitaxel and the like. Other useful antineoplastic agents include estramustine, carboplatin, cyclophosphamide, bleomycin, gemcitibine, ifosamide, melphalan, hexamethyl melamine, thiotepa, cytarabin, idatrexate, trimetrexate, dacarbazine, L-asparaginase, camptothecin, CPT-11, topotecan, ara-C, bicalutamide, flutamide, leuprolide, pyridobenzoindole derivatives, interferons and interleukins.

### General Synthetic Route Description

The compounds of FORMULA (I), in which R8 represents a group - C(=O)NRxRy as defined above, can be synthesized by the general route described in the scheme 1.

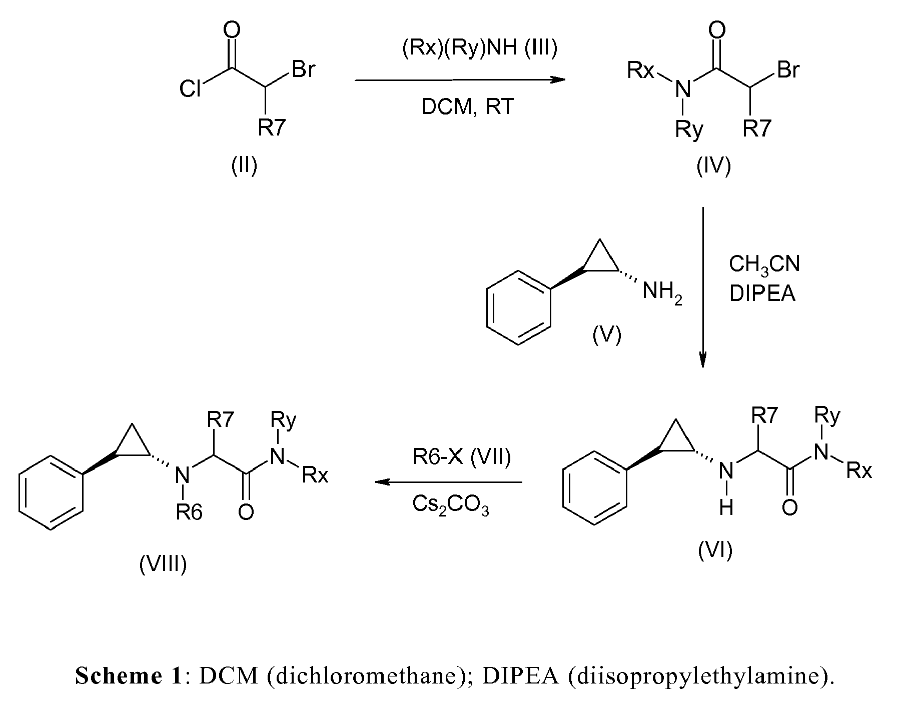

The reaction of commercially available bromoacylchlorides of formula (II) with commercially available amines of formula (III) at room temperature using dichloromethane as a solvent leads the bromoacyl derivatives of formula (IV) in high yield. These bromoacyl derivatives of formula (IV) reacts with commercially available Phenylcyclopropylamine derivatives of formula (V) using acetonitrile as a solvent and diisopropylethylamine as a base resulting in the formation of the derivatives of formula (VI), which are subject of the present invention. Alkylation of the compounds of formula (VI) using commercially available alkylating agent of formula (VII), where -X represents a good leaving group like an halogen atom, and cesium carbonate as a base results in the formation of the derivatives of formula (VIII) which are also subj ect of the present invention as defined above.

On the other hand, the compounds of formula (I), where R8 is defined as - C(OO)Rz can be synthesized in an analogous manner as described in scheme 2.

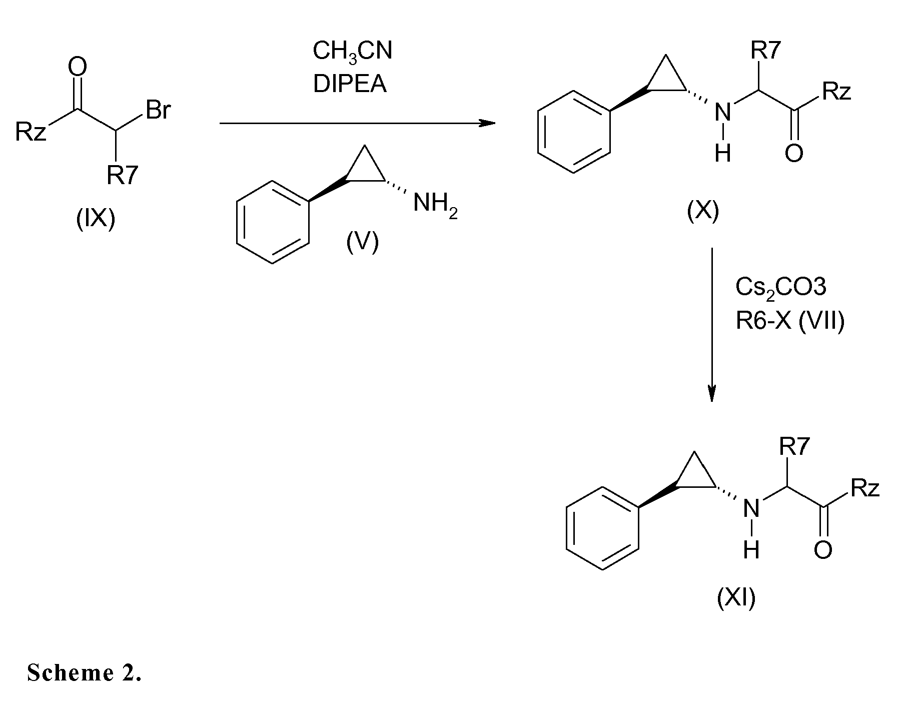

The reaction of commercially available bromoesters and bromoketones of formula (IX) with with commercially available Phenylcyclopropylamine derivatives of formula (V) using acetonitrile as a solvent and diisopropylethylamine as a base resulting in the formation of the derivatives of formula (X), which are subject of the present invention. Alkylation of the compounds of formula (VI) using commercially available alkylating agent of formula (VII), where -X is a good leaving group like halogen, and cesium carbonate as a base results in the formation of the derivatives of formula (XI) which are also subject of the present invention as defined above.

### EXAMPLES

### Example 1: N-cyclopropyl-N-methyl-trans-2-(phenylcyclopropylamino) acetamide

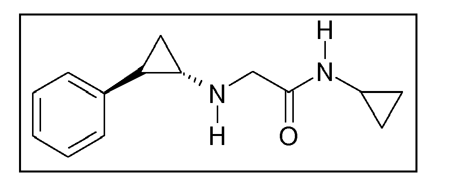

Bromoacetylchloride (450 mg, 3 mmol) was dissolved in acetonitrile (15 mL) and the solution was cooled to 0°C. To this solution cycloproylamine (170 mg, 3 mmol) was added and the mixture stirred at room temperature for 2h. To this solution trans-phenylcyclopropyl amine hydrochloride (0.50 g, 2.95 mmol) and diisoproylethylamine (1.25 mL, 7.37 mmol) were successively added and the mixture stirred at room temperature for 16h. The mixture was then concentrated *in vacuo* and the residue was purified by flash column chromatography (DCM:MeOH, 25:1) to afford the titled compound as a white solid (0.21g, 59%).
1H NMR d (ppm): 0.41 (m, 2H), 0.77 (m, 2H), 1.03 (m, 2H), 1.98 (m, 3H), 2.40 (m, 1H), 2.78 (m,1H), 3.38 (s, 2H), 6.99 (m, 3H), 7.22 (m, 3H).

The following compounds can be synthesized following the method described for example 1 using the corresponding starting materials.

### Example 2: 2-trans-2-(phenylcyclopropylamino) acetamide

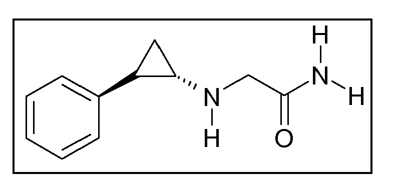

1HNMR (ppm): 1.05 (m, 2H), 1.92 (m, 2H), 2.45 (m,1H), 3.41 (s, 2H), 5.58 (bd, 1H), 6.78 (bs, 1H), 7.05 (m, 2H), 7.22 (m, 3H).

### Example 3: 2-trans-2-(Phenylcyclopropylamino)propanamide

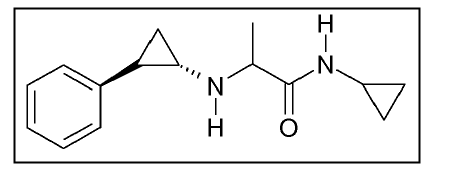

1HNMR (ppm): 0.42 (m, 2H), 0.78 (m, 2H), 1.05 (m, 2H), 1.25 (d, 3H), 1.92(m, 2H), 2.28 (m, 1H), 2.68 (m,1H), 3.27 (m, 1H), 7.22 (m, 6H).

### Example 4: 2-trans-2-(Phenylcyclopropylamino)-N-prop-2-ynyl acetamide

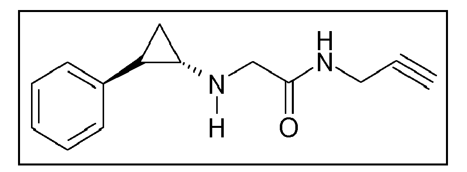

1HNMR (ppm): 1.05 (m, 2H), 1.96 (m, 2H), 2.24 (m,1H), 2.41 (m,1H), 3.42 (s, 2H), 4.08 (m, 2H), 7.01 (m, 2H), 7.22 (m, 3H).

### Example 5: N-isopropyl-trans-2-(phenylcyclopropylamino)acetamide

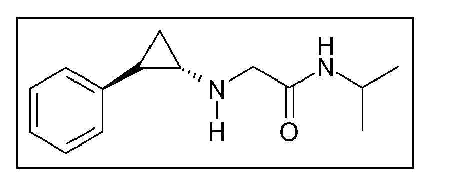

1HNMR (ppm): 1.15 (m, 8H), 1.95 (m, 2H), 2.05 (bs, 1H), 2.40 (m, 1H), 3.38 (s,2H), 4.10 (m, 1H), 6.78 (bs, 1H), 7.05 (m, 2H), 7.22 (m, 3H).

### Example 6: N-tert-butyl-trans-2-(phenylcyclopropylamino)acetamide

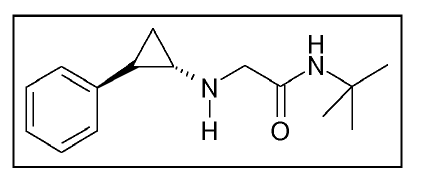

1HNMR (ppm): 1.17 (m, 2H), 1.36 (s,3H), 1.95 (m, 1H), 2.45 (m, 1H), 3.30 (s, 2H), 6.78 (bs, 1H), 7.05 (m, 2H), 7.22 (m, 3H).

### Example 7: N-morpholinyl-trans-2-(phenylcyclopropylamino) acetamide

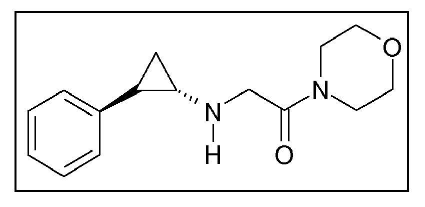

1HNMR (ppm): 1.15 (m, 2H), 1.92 (m, 1H), 2.45 (m,1H), 3.41 (m, 2H), 3.45 (s, 2H), 3.65 (m, 8H), 7.05 (m, 2H), 7.22 (m, 3H).

### Example 8: N-p-fluorophenyl-trans-2-(phenylcyclopropylamino) acetamide

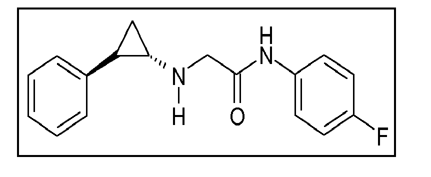

1HNMR (ppm): 1.17 (m, 2H), 2.05 (m, 1H), 2.45 (m, 1H), 3.48 (s,2H), 7.05 (m, 4H), 7.22 (m, 4H), 7.56 (m, 2H), 8.89 (bs, 1H).

### Example 9: 2-trans-2-(Phenylcyclopropylamino)propanamide

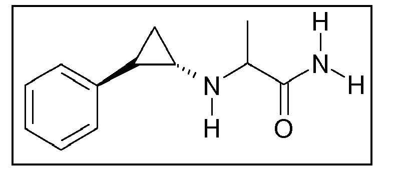

1HNMR (ppm): 1.05 (m, 2H), 1.92 (m, 2H), 2.45 (m,1H), 3.41 (s, 2H), 5.58 (bd, 1H), 6.78 (bs, 1H), 7.05 (m, 2H), 7.22 (m, 3H).

### Example 10: Methyl-2-trans-2-(phenylcyclopropylamino)propanoate

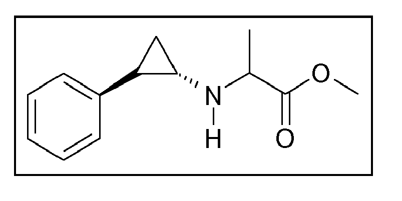

1HNMR d (ppm): 1.05 (m, 2H), 1.28 (m, 3H), 1.90 (m, 3H), 2.42 (m, 1H), 3.38 (m,1H), 5.68 (bs, 1H), 6.8 (bs, 1H), 7.00 (m, 2H), 7.22 (m, 3H)

### Example 11: N-cyclopropyl-N-methyl-trans-2-(phenylcyclopropyl amino)acetamide

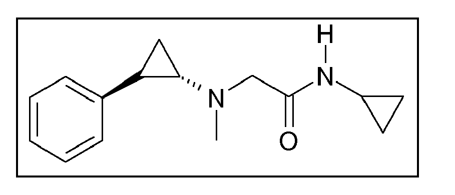

To a solution of N-cyclopropyl-N-methyl-trans-2-(phenylcyclopropyl amino)acetamide (example 1, 240 mg, 1 mmol) in acetonitrile (10 mL), methyl iodide (140 mg, 1mmol) was added and the solution was refluxed for 1h. The mixture was then concentrated *in vacuo* and the residue was purified by flash column chromatography (DCM:MeOH, 10:1) to afford the titled compound as a white solid (200 mg, 57%).

The following compounds can be synthesized following the method described for example 11 using the corresponding example and alkylating agent as starting materials.

### Example 12: N-methyl-trans-2-(phenylcyclopropylamino)acetamide

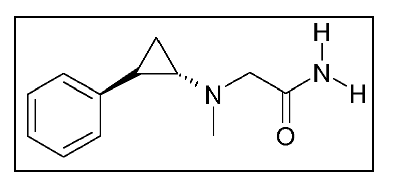

1HNMR d (ppm): 1.05 (m, 2H), 2.02 (m, 2H), 2.42 (s,3H), 3.41 (s, 2H), 5.78 (bd, 1H), 6.88 (bs, 1H), 7.01 (m, 2H), 7.22 (m, 3H).

### Example 13: N-methyl-trans-2-(Phenylcyclopropylamino)propanamide

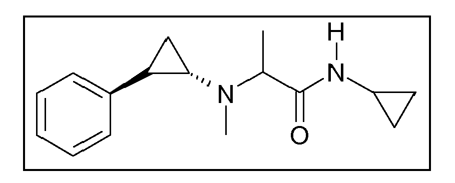

1HNMR (ppm): 0.42 (m, 2H), 0.78 (m, 2H), 1.05 (m, 2H), 1.25 (d, 3H), 1.92(m, 2H), 2.28 (m, 1H), 2.68 (m,1H), 3.27 (m, 1H), 3.34 (s, 3H), 7.22 (m, 6H).

### Example 14: N-tert-butyl-trans-2-benzyl(phenylcyclopropyl amino)acetamide

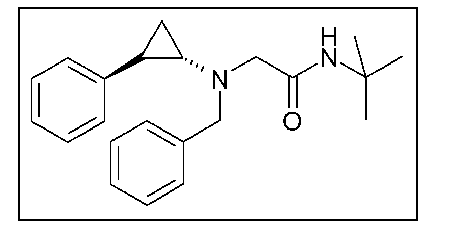

1HNMR (ppm): 1.05 (m, 2H), 1.22 (s, 9H), 1.97 (m, 2H), 2.25 (m,1H), 3.21 (m, 2H), 3.77 (m, 2H), 6.93 (m, 3H), 7.22 (m, 8H)

### Example 15: Biological Assays

The compounds of the invention can be tested for their ability to inhibit LSD1. The ability of the compounds of the invention to inhibit LSD1 can be tested as follows. Human recombinant LSD1 protein was purchased from BPS Bioscience Inc. In order to monitor LSD1 enzymatic activity and/or its inhibition rate by our inhibitor(s) of interest, di-methylated H3-K4 peptide (Millipore) was chosen as a substrate. The demethylase activity was estimated, under aerobic conditions, by measuring the release of H₂O₂ produced during the catalytic process, using the Amplex® Red peroxide/peroxidase-coupled assay kit (Invitrogen).

Briefly, a fixed amount of LSD1 was incubated on ice for 15 minutes, in the absence and/or in the presence of various concentrations of inhibitor (from 0 to 75 µM, depending on the inhibitor strength). Tranylcypromine (Biomol International) was used as a control for inhibition. Within the experiment, each concentration of inhibitor was tested in triplicate. After leaving the enzyme interacting with the inhibitor, 12.5 µM of di-methylated H3-K4 peptide was add to each reaction and the experiment was left for 1 hour at 37C in the dark. The enzymatic reactions were set up in a 50 mM sodium phosphate, pH 7.4 buffer. At the end of the incubation, Amplex® Red reagent and horseradish peroxidase (HPR) solution were added to the reaction according to the recommendations provided by the supplier (Invitrogen), and leaved to incubate for 30 extra minutes at room temperature in the dark. A 1 µM H₂O₂ solution was used as a control of the kit efficiency. The conversion of the Amplex® Red reagent to resorufin due to the presence of H₂O₂ in the assay, was monitored by fluorescence (excitation at 540 nm, emission at 590 nm) using a microplate reader (Infinite 200, Tecan). Arbitrary units were used to measure level of H2O2 produced in the absence and/or in the presence of inhibitor.

The maximum demethylase activity of LSD1 was obtained in the absence of inhibitor and corrected for background fluorescence in the absence of LSD1. The Ki of each inhibitor was estimated at half of the maximum activity.

A number of the compounds of the invention were tested for their ability to inhibit LSD1 and where found to have Ki values lower than 100 µM, including the compounds in examples 3, 6, 7, 8, 11, and 13. Compound of examples 1, 2, 4, 5, 9, 10, and 12 were found to have Ki values for LSD1 of less than 10 µm

All publications and patent applications mentioned in the specification are indicative of the level of those skilled in the art to which this invention pertains. All publications and patent applications are herein incorporated by reference to the same extent as if each individual publication or patent application was specifically and individually indicated to be incorporated by reference. The mere mentioning of the publications and patent applications does not necessarily constitute an admission that they are prior art to the instant application.

Although the foregoing invention has been described in some detail by way of illustration and example for purposes of clarity of understanding, it will be obvious that certain changes and modifications may be practiced within the scope of the appended claims.

## Claims

1. A compound of Formula I: 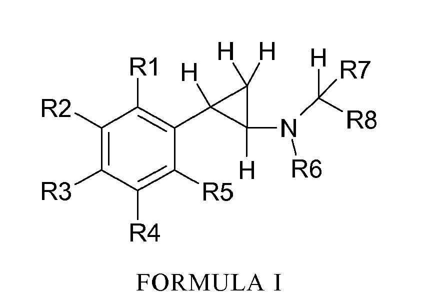 wherein
each of R1-R5 is independently selected, may be optionally substituted, and is chosen from -H, halo, alkyl, alkoxy, haloalkyl, haloalkoxy, -L-aryl, -L-heteroaryl, -L-heterocycle, -L-carbocycle, acylamino, acyloxy, alkylthio, alkynyl, amino, aryl, arylalkyl, arylalkenyl, arylalkynyl, arylalkoxy, aryloxy, arylthio, cyano, cyanato, haloaryl, hydroxyl, heteroaryloxy, heteroarylalkoxy, isocyanato, isothiocyanato, nitro, sulfinyl, sulfonyl, sulfonamide, thiocarbonyl, thiocyanato, trihalomethanesulfonamido, O-carbamyl, N-carbamyl, O-thiocarbamyl, N-thiocarbamyl, and C-amido;
R6 is chosen from -H and alkyl;
R7 is chosen from -H, alkyl, and cycloalkyl;
R8 is chosen from -C(=O)NRₓR_{y} and -C(=O)R_{z};
Rₓ is chosen from -H, alkyl, alkynyl, alkenyl, -L-carbocycle, -L-aryl, -L-heterocycle, all of which can be optionally substituted (except -H);
R_{y} is chosen from -H, alkyl, alkynyl, alkenyl, -L-carbocycle, -L-aryl, -L-heterocycle, all of which can be optionally substituted (except -H);
R_{z} is chosen from -H, alkoxy, -L-carbocyclic, -L-heterocyclic, -L-aryl, wherein the aryl, heteroaryl, heterocycle, or carbocycle is optionally substituted;
each L can be saturated, partially saturated, or unsaturated, and is independently chosen from -(CH₂)ₙ-(CH₂)ₙ-, -(CH₂)ₙC(=O)(CH₂)ₙ-, - (CH₂)ₙC(=O)N(CH₂)ₙ-, - (CH₂)ₙNC(=O)O(CH₂)ₙ-, -(CH₂)ₙNC(=O)N(CH₂)ₙ-, - (CH₂)ₙNC(=S)S(CH₂)ₙ-, - (CH₂)ₙOC(=O)S(CH₂)ₙ-, -(CH₂)ₙNH(CH₂)ₙ-, - (CH₂₎ₙO(CH₂)ₙ-, -(CH₂)ₙS(CH₂)ₙ-, and -(CH₂)ₙNC(=S)N(CH₂)ₙ-, where each n is independently chosen from 0, 1 ,2, 3, 4, 5, 6, 7, and 8, and wherein each carbon and/or nitrogen can be optionally substituted with one or more substituents independently chosen from hydroxyl, halo, alkoxy, alkyl, amino, cycloalkyl, -NRₐR_{b}, -NSO₂R_{c}, -NC(=O)NRₐR_{b}, heteroaryl, aryl, carbocycle, and heterocyclic; wherein -Rₐ and -R_{b} are independently chosen from hydro, alkyl, and -C(=O)OR_{c}; and wherein R_{c} is an alkyl group (e.g., methyl, ethyl, propyl, isopropyl, butyl, isobutyl, or tert-butyl); and pharmaceutically acceptable salts thereof.

2. The compound of claim 1 wherein each of R1-R5 is independently chosen from -H, halo, C1-C4 alkyl, C-C4 alkoxyl, C1-C4 haloalkyl, -OCH₂(phenyl), and C1-C4 haloalkoxy.

3. The compound of claim 1 wherein each of R1-R5 is independently chosen from -H, halo, -OCH₂(phenyl) and -CF₃.

4. The compound of claim 1 wherein each of R1-R5 is -H.

5. The compound of claim 1 wherein R6 is -H or a C1-C4 alkyl.

6. The compound of claim 1 wherein R7 is -H or a C1-C4 alkyl.

7. The compound of claim 1 wherein each L is independently chosen from a bond,
-CH₂-, -CH₂CH₂-, and -CH₂CH₂CH₂-.

8. The compound of claim 1 wherein L is chosen from a bond and -CH₂-.

9. The compound of claim 1 wherein Rₓ if present, is chosen from -C1-C6 alkyl, C2-C6 alkenyl, C2-C6 alkynyl, -L-cycloalkyl, -L-aryl, -L-heterocycle, all of which can be optionally substituted except -H.

10. The compound of claim 1 wherein Rₓ is chosen from -H, -CH(CH₃)₂, -C(CH₃)₃, -CH₂C≡CH, -CH₂CH=CH₂, cyclopropyl, cyclobutyl, cyclopentyl, cyclohexyl, and -CH₂(phenyl) wherein the cycloalkyl and phenyl groups can be optionally substituted.

11. The compound of claim 1 wherein R_{y} if present, is chosen from -C1-C6 alkyl, C2-C6 alkenyl, C2-C6 alkynyl, -L-cycloalkyl, -L-aryl, -L-heterocycle, wherein the cycloalkyl, aryl, and heterocycle can be optionally substituted.

12. The compound of claim 1 wherein R_{y} is chosen from -H, -CH(CH₃)₂, -C(CH₃)₃, -CH₂C≡CH, -CH₂CH=CH₂, cyclopropyl, cyclobutyl, cyclopentyl, cyclohexyl, and -CH₂(phenyl), wherein the cycloalkyl and phenyl groups can be optionally substituted.

13. The compound of claim 1 wherein R_{z} if present, is an optionally substituted -L-heterocycle. In a more specific aspect, R_{z} is optionally substituted and chosen from N-methylpiperazinyl, morpholinyl, and piperidinyl. In a more specific aspect, R_{z} is chosen from N-methylpiperazinyl, morpholinyl, and piperidine.

14. A pharmaceutical composition comprising a compound of claim 1 and a pharmaceutically acceptable carrier.

15. A method of treating cancer comprising administering to a patient a therapeutically effective amount of a compound according to any of claims 1-13.

16. A method of treating cancer comprising administering to a patient a therapeutically effective amount of a compound according to any of claims 1-13.

17. A compound according to any of claims 1-13 where said compound has the trans configuration around the cyclopropyl ring.

18. A method of inhibiting LSD1 comprising administering to a patient an LSD1 inhibiting effective amount of a compound according to any one of claims 1-13.

19. A compound according to claim 1 wherein said compound is chosen from N-cyclopropyl-N-methyl-trans-2-(phenylcyclopropylamino) acetamide, 2-trans-2-(phenylcyclopropylamino) acetamide, 2-trans-2-(Phenylcyclopropylamino)propanamide, 2-trans-2-(Phenylcyclopropylamino)-N-prop-2-ynyl acetamide, N-isopropyl-trans-2-(phenylcyclopropylamino)acetamide, N-tert-butyl-trans-2-(phenylcyclopropylamino)acetamide, N-morpholinyl-trans-2-(phenylcyclopropylamino) acetamide, N-p-fluorophenyl-trans-2-(phenylcyclopropylamino) acetamide, 2-trans-2-(Phenylcyclopropylamino)propanamide, Methyl-2-trans-2-(phenylcyclopropylamino)propanoate, N-cyclopropyl-N-methyl-trans-2-(phenylcyclopropyl amino)acetamide, N-methyl-trans-2-(phenylcyclopropylamino)acetamide, N-methyl-trans-2-(Phenylcyclopropylamino)propanamide, and N-tert-butyl-trans-2-benzyl(phenylcyclopropyl amino)acetamide, and pharmaceutically acceptable salts thereof.
